# EUROPÄISCHE PATENTSCHRIFT

(11) **EP 1 656 161 B1**
(45) Veröffentlichungstag und Bekanntmachung des Hinweises auf die Patenterteilung: **17.09.2008**
(21) Anmeldenummer: 04762525.6
(22) Anmeldetag: 28.07.2004
(51) Int. Cl.: A61L 9/14, A61L 9/22

(54) **VERFAHREN ZUM VERTEILEN VON FLÜSSIGEN DUFTSTOFFEN UND VORRICHTUNG ZUR DURCHFÜHRUNG DES VERFAHRENS**
METHOD FOR THE DISTRIBUTION OF LIQUID FRAGRANCES AND DEVICE FOR CARRYING OUT SAID METHOD
PROCEDE DE DISTRIBUTION DE PARFUMS LIQUIDES, ET DISPOSITIF POUR LA MISE EN OEUVRE DE CE PROCEDE

(30) Priorität: 28.07.2003 DE 10334591
(43) Veröffentlichungstag der Anmeldung: 17.05.2006
(73) Patentinhaber: International Flavors & Fragrances, Inc., New York New York 10019 (US)
(72) Erfinder: BARTELS, Frank, 45527 Hattingen (DE)
(74) Vertreter: Griepenstroh, Jörg
(86) Internationale Anmeldenummer: PCT/DE2004/001680
(87) Internationale Veröffentlichungsnummer: WO 2005/011761

(56) Entgegenhaltungen:
- DE-A- 10 145 954
- US-A- 5 382 410

## Beschreibung

Die Erfindung betrifft ein Verfahren zum Verteilen von flüssigen Duftstoffen mit den Merkmalen des Oberbegriffs des Patentanspruchs 1 sowie eine Vorrichtung zur Durchführung des Verfahrens gemäß den Merkmalen im Oberbegriff des Patentanspruchs 11.

Duftstoffe und Duftstoffzusammensetzungen, die in einen Raum abgegeben werden, dienen nicht nur zur Verbesserung des Raumklimas, sondern können auch zur biologischen Behandlungen und im kommunikativen Bereich eingesetzt werden, beispielsweise um eine höhere Aufmerksamkeit zu erreichen. Es ist bekannt, das ein höheres Wirkungsniveau hinsichtlich der Aufmerksamkeit erreicht wird, wenn audiovisuelle und olfaktorische Medien gleichzeitig eingesetzt werden. Hierzu können Düfte synchron zu audiovisuellen Darstellungen freigesetzt werden und über die Umgebungsluft als Trägermedium verbreitet werden.

Es ist bekannt, aromatische Essenzen und andere Flüssigkeiten auf unterschiedliche Art und Weise in die Gasphase zu überführen. Die grundlegenden physikalischen Prinzipien sind das Vernebeln und das Verdampfen. Unter Verdampfen versteht man den allmählichen Übergang einer Flüssigkeit in den gasförmigen Zustand. Die Moleküle jeder Flüssigkeit befinden sich in ständiger Bewegung zwischen flüssigem und gasförmigem Zustand. Ihre Durchschnittsgeschwindigkeit ist umso größer, je höher die Temperatur ist. Je wärmer die Flüssigkeit ist, desto größer wird der Verdampfungsdruck, d.h. desto mehr Flüssigkeit verdampft. Ist der Verdampfungsdruck gleich dem Umgebungsluftdruck, so beginnt die Flüssigkeit zu sieden. Da immer einige Moleküle deutlich schneller sind als andere, können sie die Flüssigkeit gasförmig verlassen. Die Verdunstung oder Verdampfung hat den Nachteil, dass sich die Duftstoffzusammensetzungen mit der Zeit verändern. Duftstoffe bestehen in der Regel aus einem Gemisch unterschiedlich flüchtiger Stoffe. Leicht flüchtige Duftnoten bestimmen den Angeruch oder die Kopfnote, schwer flüchtige den Nachgeruch oder die Basisnote eines Duftstoffes. Grundsätzlich sind Veränderungen während des Duftablaufs normal, wobei jede Duftstoffzusammensetzung während jeder Phase einen gleichen Grundcharakter beibehalten sollte. Bei der Verdunstung oder Verdampfung wird die Veränderung des Duftablaufs unter Umständen derart beschleunigt, so dass die zuerst wahrnehmbare Kopfnote einer Duftstoffzusammensetzung zuerst verloren geht, während zunehmend schwer flüchtige Bestandteile der Duftstoffzusammensetzung verbleiben. Dadurch verändert sich der Duft häufig nachteilig, so dass eine insbesondere im kommunikativen Bereich eingesetzte Raumbeduftung ihre Wirkung einbüßen kann. Dies gilt umso mehr, je höher die thermische Belastung des Duftstoffes ist. Bei höheren Temperaturen kann zwar ein größerer Volumenstrom verdampft werden, allerdings leidet hierunter oft die Harmonie der Duftstoffzusammensetzung.

Alternativ zur Verdampfung besteht die Möglichkeit der Duftstoffzerstäubung, bei welcher ein feiner Nebel aus einer Vielzahl kleiner Tropfen erzeugt wird. Die Tropfenbildung kann unter Druck erfolgen, wobei der Duftstoff beispielsweise durch ein Treibmittel in einem lokalen Druckspeicher vorgespannt wird. Es ist ferner bekannt, zur Ausbringung des Duftstoffs Piezoaktoren zu verwenden, die den Flüssigkeitsstrom durch einzelne Düsen pressen.

Bei bekannten Anordnungen zur Duftstoffanbringung wird zum, Teil auch der sogenannte Kapillareffekt bewusst genutzt, um den Duftstoff zu befördern. Der Kapillareffekt führt jedoch dazu, dass immer eine gewisse Menge des Duftstoffs mit der Atmosphäre in Kontakt steht. Über diese Kontaktfläche findet eine kontinuierliche Verdampfung der Kopfnote des Duftstoffs statt, so dass sich die Duftnote zeitabhängig unkontrolliert verändert.

Durch die EP-B-0574547 ist eine Vorrichtung zum Erzeugen elektrostatisch geladener Aerosole und/oder Dämpfe bekannt, bei welcher eine poröse Kapillareinheit mit mehreren Faserfäden einen Duftstoff von einem Reservoir zu einer Abgabe überführt. Hierbei wird die Flüssigkeit durch eine Hochspannungsgleichstromquelle elektrostatisch aufgeladen und kann in Form von Aerosolen und/oder Dämpfen von den sich frei erstreckenden Spitzen der Kapillareinheit abgegeben werden. Die Flüssigkeit wird durch die Kapillarwirkung ständig zum oberen Ende der als Docht wirkenden Kapillareinheit transportiert, selbst wenn keine Spannung anliegt. Die Materialeigenschaften der Kapillareinheit haben bei beliebig eingestellter Spannung einen signifikanten Einfluss auf die Effizienz der Dampf- und/oder Aerosolabgabe. Die Porösität, die elektrische Leitfähigkeit und die Form der Materialien bestimmen das Dampf-Aerosol-Verhältnis sowie auch die Menge an erzeugten Luftionen. Insbesondere bei sehr langen Kapillaren zwischen einer ggf. vorgesehenen Flüssigkeitspumpe und dem Abgabeende der Kapillareinheit erfolgt je nach Flüchtigkeit der verwendeten Duftstoffe ein mehr oder weniger umfangreicher Abdampfprozess einzelner Duftstoffe. Die Steuerung der Dampf- und/oder Aerosolabgabe ist dadurch relativ kompliziert.

Aus der DE 101 45 954 A ist ein universelles Duftausbringungssystem für den stationären Einsatz bekannt. Das Duftausbringungssystem umfasst einen oder mehrer Duftstoffbehälter, aus denen über ein oder mehrere Röhrchen eine oder mehrere Pumpen, vorzugsweise in Mikrosystemtechnik, Duftstoffe zu einem oder mehreren Verdampfungselementen befördern. Ein Luftstromgenerator mit einem optional beheizbaren Strömungsrohr befördert den verdampften Duftstoff aus dem System zu einer oder mehreren Zielpersonen oder in den Raum. Eine optional von außen ansteuerbare Elektronik steuert die Pumpen und die Verdampfungselemente sowie den Luftstromgenerator.

Hiervon ausgehend liegt der Erfindung die Aufgabe zugrunde, ein leicht steuerbares Verfahren zum Verteilen von flüssigen Duftstoffen aufzuzeigen, bei welchen ein kontrolliertes Flüssigkeitsvolumen in ein Aerosol überführt wird, wobei eine unkontrollierte Tröpfchenbildung an der Abgabeeinheit verhindert und die Möglichkeit einer unerwünschten vorzeitigen Verdampfung von flüchtigen Duftstoffkomponenten weitestgehend unterbunden wird.

Eine weitere Aufgabe der Erfindung ist es, eine Vorrichtung aufzuzeigen, mit welcher dieses Verfahren besonders günstig durchgeführt werden kann.

Die erste Aufgabe ist bei einem Verfahren mit den Maßnahmen des Patentanspruchs 1 gelöst.

Das erfindungsgemäße Verfahren zeichnet sich dadurch aus, dass ein der Abgabeeinheit vorgeschaltetes Absperr- und Stellorgan einer Zuflussleitung zeitversetzt zu der Hochspannungseinheit aktiviert wird, wobei ein innerhalb der Zuflussleitung zwischen dem Absperr- und Stellorgan und der Abgabeeinheit vorhandener Duftstoff mengenmäßig reduziert wird. Eine mengemäßige Reduzierung im Sinne der Erfindung ist nicht als "Eindickung" einer Flüssigkeit durch Zufuhr thermischer Energie zu verstehen, sondern als gesteuertes Vernebeln der Restmenge durch Anwendung des Elektrosprayverfahren. Das Elektrosprayverfahren hat den Vorteil, dass die Partikelgröße der erzeugten Aerosole sehr gleichmäßig und vor allem sehr klein ist. Die Partikelgröße kann kleiner sein als ein Mikrometer. Die aus einer Kapillarspitze der Abgabeeinheit austretenden hoch geladenen Tröpfchen gleicher Polarität stoßen sich gegenseitig ab und bilden einen extrem feinen Nebel, der nur in einem sehr geringen Abstand zur Abgabeeinheit sichtbar ist. Bereits nach wenigen Zentimetern ist der Duftstoffnebel für das menschliche Auge nicht mehr erkennbar, da er in die Gasphase übergegangen ist. Mit dem Elektrosprayverfahren können zudem sehr kleine Mengen eines Duftstoffs kontrolliert abgegeben werden.

Die bei diesem Verfahren vorgesehene Reduzierung der Duftstoffmenge kann sogar so weit gehen, dass der Duftstoff im Bereich der Abgabeeinheit vollständig ausgetragen wird, so dass im wesentlichen kein Duftstoff mehr in Kontakt mit der Atmosphäre steht. Diese Voraussetzung kann sowohl dann erfüllt sein, wenn die Vorrichtung in Betrieb genommen wird als auch dann, wenn die Ausbringung des Duftstoffs beendet wird. Dies kann im ersten Fall dadurch erreicht werden, dass bei der Verteilung des Duftstoffs zunächst die Hochspannungseinheit aktiviert wird und anschließend das Absperr- und Stellorgan geöffnet wird (Patentanspruch 2). Hierdurch ist sichergestellt, dass sich an der Abgabeeinheit kein unkontrolliert wachsender Tropfen bilden kann, der erst beim nachträglichen Anschalten der Hochspannungseinheit abgebaut werden muss. Würde die Hochspannungseinheit erst später angeschaltetet werden, müsste der aus der Abgabeeinheit austretende Tröpfchenstrahl den anhaftenden Tropfen durchdringen und mitreißen. Hierdurch entstehen unterschiedliche Tröpfchengrößen, die zu einer unterschiedlichen Duftentfaltung führen können. Bei sehr großen Tröpfchen besteht sogar die Gefahr, dass sich diese, bedingt durch die Schwerkraft, im unmittelbaren Umgebungsbereich der Abgabeeinheit ablagern, was auch nach Beendigung des Abgabevorgangs eine unkontrollierte Verdampfung des Duftstoffs zur Folge hätte. Wird hingegen die Hochspannungseinheit zuerst angeschaltet und anschließend das Absperr- und Stellorgan geöffnet, ist sichergestellt, dass der Volumenstrom des Duftstoffs an der Abgabeeinheit zunächst geringer ist als am Absperr- und Stellorgan, so dass eine hinsichtlich der Tropfengröße und Ausbringungsmenge kontrollierte und dosierte Aerosolabgabe möglich ist.

Wird die Verteilung des Duftstoffes zeitweise unterbrochen oder auch vollständig beendet, ist es wichtig, dass zunächst das Absperr- und Stellorgan geschlossen wird, um die Zuführung des Duftstoffes zu der Abgabeeinheit zu unterbrechen. Erst nachdem das Absperr- und Stellorgan bzw. die Zuflussleitung geschlossen ist, wird gemäß den Merkmalen des Patentanspruchs 3 die Hochspannungseinheit deaktiviert. Diese zeitliche Reihenfolge gewährleistet, dass die zwischen dem Absperr- und Stellorgan und der Abgabeeinheit verbleibende Restmenge des Duftstoffes aktiv ausgetragen wird, was zu einer weitestgehenden Entleerung dieses Längenabschnitts der Zuflussleitung führt. Wichtig ist dabei, dass das Absperr- und Stellorgan bis zur erneuten Abgabe eines Duftstoffes geschlossen bleibt, damit nicht unkontrolliert Duftstoff durch den Kapillareffekt in Richtung zur Abgabeeinheit gefördert wird.

Die Vorteile des erfindungsgemäßen Verfahrens kommen insbesondere dann zum Tragen, wenn mehrere verschiedene Duftstoffe durch die Vorrichtung gleichzeitig oder zeitversetzt abgegeben werden sollen, um beispielsweise im Bereich der Duftkommunikation bestimmte Kommunikationsleistungen und/oder -intensitäten zu erzielen. Während die Intensität des Duftstoffes im wesentlichen von der ausgebrachten Duftstoffmenge abhängt, ist die Kommunikationsleistung von der Duftnote und der Reihenfolge der Duftnoten abhängig. Daher ist eine exakte Steuerbarkeit oder sogar Regelbarkeit der erfindungsgemäßen Vorrichtung von größter Bedeutung.

Es sind Anwendungsfälle vorstellbar, bei denen gleichzeitig mehrere unterschiedliche Duftstoffe ausgebracht werden, wobei die gewünschte Duftwirkung erst durch die Überlagerung der Duftstoffe entsteht. Voraussetzung hierbei ist, dass die Duftstoffe miteinander harmonieren und dass die einzelnen Duftstoffmengen exakt aufeinander abgestimmt sind.

Es ist aber auch vorstellbar, dass durch eine zeitversetzte Abgabe einzelner Duftstoffe in gewisser Weise eine Duftgeschichte erzählt wird, bei welcher nicht in jedem Fall eine Überlagerung der einzelnen Duftstoffe angestrebt wird. Falls zeitlich aufeinander folgende Duftstoffe nicht miteinander harmonisieren, ist es wünschenswert, Überlagerungen und Vermischungen weitestgehend zu vermeiden. Dies gelingt mit dem erfindungsgemäßen Verfahren dadurch, dass Restmengen des zuerst ausgebrachten Duftstoffes soweit wie möglich reduziert werden, so dass auch die schwer flüchtige Basisnote dieses Duftstoffes vollständig eliminiert wird und nicht die Kopfnote des nachfolgend ausgebrachten Duftstoffs überlagert.

Die Vorteile der Erfindung kommen auch dann zum Tragen, wenn über eine einzige Abgabeeinheit unterschiedliche Duftstoffe abgegeben werden sollen. Wenn beispielsweise ein erster Duftstoff in einem der Vorrichtung lösbar zugeordneten, austauschbaren Reservoir bevorratet wird, welcher nach der Entleerung gegen ein Reservoir mit einem anderen Duftstoff ausgetauscht wird, soll bei einem solchen Duftwechsel selbstverständlich der neu eingesetzte Duftstoff sofort abgegeben werden können, ohne sich dabei mit Restmengen alten Duftstoffs zu vermengen.

Nach Patentanspruch 4 ist vorgesehen, dass an die Abgabeeinheit eine elektrische Hochspannung in einem Bereich von 0,5 kV bis 25 kV angelegt wird.

Vorzugsweise liegt die elektrische Hochspannung in einem Bereich von 1,5 bis 6 kV (Patentanspruch 5). Die Hochspannung kann auf engstem Bauraum durch eine Hochspannungskaskade erzeugt werden. Da bereits eine geringe Eingangsspannung zur Erzeugung der Hochspannung ausreichend ist, kann eine entsprechende Vorrichtung batteriebetrieben, d.h. netzunabhängig zum Einsatz kommen.

Als besonders in der Höhe vorteilhaft wird es angesehen, wenn die elektrische Hochspannung konstant gehalten wird, während der Volumenstrom des Duftstoffs über das Absperr- und Stellorgan gesteuert wird (Patentanspruch 6). Auf diese Weise kann der Steuer- bzw. der Regelaufwand bei der verwendeten Vorrichtung erheblich reduziert werden, da die Ausbringungsmenge ausschließlich durch die Stellung des Absperr- und Stellorgans beeinflusst wird. Die elektrische Hochspannung braucht lediglich zeitversetzt an- oder ausgeschaltet werden, ohne dass dies Einfluss auf die Ausbringungsmenge hätte. Bei der Hochspannung kann es sich um eine Gleichspannung handeln. Die Polarität kann zwischen zwei Sprayvorgängen allerdings auch wechseln.

Nach Patentanspruch 7 ist vorgesehen, dass das Verändern des Volumenstroms mit Hilfe einer Mikropumpe erfolgt. Mikropumpen als solche sind bekannt. Sie können aus Kunststoff, insbesondere Polycarbonat, hergestellt sein und werden von einem piezokeramischen Aktuator angetrieben, der auf eine Membran einwirkt. Die Förderrate kann durch die Amplitude des piezokeramischen Aktuators moduliert werden. Mikropumpen sind einfach in fluidische Systeme integrierbar und kostengünstig herstellbar.

Es wird als besonders vorteilhaft angesehen, wenn das maximale Fördervolumen der Mikropumpe kleiner oder gleich der Ausbringungskapazität der Abgabeeinheit eingestellt wird (Patentanspruch 8). Hierdurch ist sichergestellt, dass die Abgabeeinheit in jedem Fall den von der Mikropumpe geförderten Volumenstrom in der Weise bewältigen kann, dass eine gleichbleibende Tröpfchengröße und eine homogene Vernebelung des Duftstoffs erzielt wird.

In der Ausgestaltung des Patentanspruchs 9 wird jeder Abgabeeinheit ein unterschiedlicher Duftstoff zugeführt, wobei die Duftstoffe separat zeit- und/oder volumengesteuert in Aerosole überführt werden. Bei diesem Verfahren können unterschiedliche Duftstoffe durch zeit- und/oder volumenabhängige Steuerung gemischt oder in einer vorbestimmten zeitlichen Reihenfolge gezielt ausgebracht werden. Die Steuerung oder Regelung der Duftausbringung kann programmgesteuert, beispielsweise in Wellenform, erfolgen. Durch die Möglichkeit, gleichzeitig mehrere Duftstoffe auszubringen, ist eine entsprechende Vorrichtung ausgesprochen flexibel einsetzbar. Mit dem Elektrosprayverfahren können zudem in hoch effizienter Weise Duftöle oder Dufststoffkonzentrate in geringsten Mengen kontrolliert dosiert ausgebracht werden. Die Baugröße entsprechender Vorrichtungen ist dadurch sehr gering. Die Steuerung bzw. Regelung der verwendeten Vorrichtung kann durch einen Mikroprozessor der Steuereinheit erfolgen. Grundsätzlich ist es im Rahmen der Erfindung auch möglich, Schnittstellen zu einer externen Steuerung vorzusehen, um entweder die interne Steuerung der verwendeten Vorrichtung zu programmieren oder von der externen Steuerung aktiv in die Steuerung und Regelung der verwendeten Vorrichtung einzugreifen.

Die Steuerung oder Regelung der Vorrichtung kann in einem kontinuierlichen Automatikmodus erfolgen. Manuelle Eingriffe sind möglich. Durch einen manuellen Eingriff können beispielsweise auch unterschiedliche Automatikmodi gewählt werden. Es ist denkbar, dass durch Bedienereingriff unterschiedliche Intensitätsniveaus der Duftstoffausbringung wählbar sind, wie dies z.B. in Abhängigkeit von der Raumgröße wünschenswert sein kann. Ein hohes Intensitätsniveau entspricht dabei einem hohen Ausbringungsvolumen pro Zeiteinheit. Selbstverständlich sollte der Benutzer die Möglichkeit haben, auf Wunsch sofort einen oder mehrere Duftststoffe mit der Vorrichtung freizusetzen. Hierzu können entsprechende Betätigungseinrichtungen, beispielsweise ein oder mehrere Druckknöpfe an der Vorrichtung vorgesehen sein.

Die Betriebsdauer der verwendeten Vorrichtung ist insbesondere davon abhängig, wie viel Duftstoff innerhalb eines Reservoirs zur Verfügung gestellt wird.

In einer vorteilhafen Ausführungsform wird eine Mindestbetriebsdauer von 45 Tagen bei Batteriebetrieb angestrebt, wobei während dieser Zeit etwa 10 bis 20 Milliliter Duftstoff abgegeben werden. Die Ausbringungsmenge ist dabei stark abhängig von dem Einsatzbereich und der Anzahl der Abgabeeinheiten der verwendeten Vorrichtung. Es ist im Rahmen der Erfindung beispielsweise vorstellbar, dass ein erster Duftstoff über eine erste Abgabeeinheit in z.B. fünf Einzelstößen abgegeben wird, unmittelbar anschließend erfolgt die Abgabe eines zweiten Duftstoffs und ggf. weiterer Duftstoffe. In einem anderen Modus können zwischen den Abgaben der einzelnen Duftstoffe Pausen von z.B. 8 bis 10 Sekunden vorgesehen sein, um eine unmittelbare Vermischung der Duftstoffe zu vermeiden und um durch den zeitlichen Versatz neue Duftreize auszuüben. Nachdem eine Reihenfolge unterschiedlicher Duftstoffe mit oder ohne Unterbrechung abgegeben worden ist, kann selbstverständlich eine Pause vorgesehen sein, bevor in gleicher oder geänderter Reihenfolge erneut Duftstoffe abgegeben werden. Die Pause kann wenige Sekunden betragen. Selbstverständlich können je nach gewünschter Ausbringungsmenge auch Pausen von mehreren Minuten vorgesehen sein. Diese Pausen haben unmittelbaren Einfluss auf die Ausbringungsmenge, so dass in vorteilhafter Ausgestaltung des Erfindungsgedankens eine stufenlose Einstellung der Pausenlänge durch ein manuelles Betätigungsmittel erfolgen kann, ohne dass aufwendige Eingriffe in die Steuerung oder Regelung der Absperr- und Stellorgane erforderlich sind.

Es wird ferner als vorteilhafte Maßnahme angesehen, wenn der Duftstoff über die Zuflussleitung einem austauschbaren Duftstoffspeicher mit einer flexiblen Umhüllung entnommen wird (Patentanspruch 10). In der flexiblen Umhüllung ist der Duftstoff ohne Zutritt zur Atmosphäre aufgenommen, so dass bereits im Bereich des Duftstoffspeichers ein Verdampfen der Kopfnote des Duftstoffs ausgeschlossen werden kann. Der gesamte Duftstoff wird von dem Duftstofspeicher über die Zuflussleitung sowie das Absperr- und Stellorgan bis zur Abgabeeinheit ohne Kontakt mit der Atmosphäre transportiert, so dass der Duft bis zur Vernebelung unverfälscht beibehalten wird. Der Duftstoffspeicher kann mit einer Anzeige ausgestattet sein, anhand derer der Füllgrad und insbesondere eine zeitlich unmittelbar bevorstehende vollständige Entleerung des Duftstoffspeichers angezeigt wird. In einfachster Ausgestaltung besitzt die flexible Umhüllung zumindest einen teilweise durchsichtigen Bereich, der ggf. mit einer Skalierung versehen ist, so dass der Füllgrad unmittelbar ablesbar ist.

Je nach Ausführungsform kann die flexible Umhüllung druckbeaufschlagt sein, so dass zum einen eine vollständige Entleerung des Duftstoffspeichers gewährleistet wird und zum anderen der Volumenstrom unter einem Mindestdruck dem Absperr- und Stellorgang zugeführt wird. Bei dieser Konstellation ist es möglich, als Absperr- und Stellorgang ein den Durchfluss steuerndes oder regelndes Ventil vorzusehen, ohne dass weitere Pumpmittel erforderlich sind. Entscheidend ist, dass das Absperr- und Stellorgan in seiner Grundstellung geschlossen ist und nur zur Abgabe des Duftstoffs teilweise oder vollständig geöffnet wird. Grundsätzlich ist es bei Verwendung einer Mikropumpe jedoch ausreichend, wenn der Duftstoff im Saugbetrieb aus dem Duftstoffspeicher entnommen werden kann. Das sich verringernde Volumen im Duftstoffspeicher wird durch die Verformung der flexiblen Umhüllung durch den Umgebungsdruck ausgeglichen, so dass bei einsprechender Flexibilität der Umhüllung eine weitestgehend vollständige Entleerung des Duftstoffspeichers möglich ist. Vorzugsweise liegt die Entnahmeöffnung des Duftstoffspeichers in der natürlichen Fließrichtung des Duftstoffs, d.h. unten.

Grundsätzlich kann das Absperr- und Stellorgang Bestandteil der Mikropumpe sein. Hierbei kann beispielsweise ein in der Mikropumpe vorgesehenes Rückschlagventil, das nur eine Durchströmungsrichtung zulässt, als Absperrorgan des Absperr- und Stellorgans wirken, wobei die Stell- oder Regelfunktion über die Ansteuerung der Mikropumpe erfolgt, die unmittelbar Einfluss auf den Volumenstrom nimmt. Selbstverständlich können auch zusätzliche Absperr- und Stellorgane vorgesehen sein, die der Mikropumpe vor- und/oder nachgeschaltet sind.

Im Rahmen der Erfindung wird es sogar als möglich angesehen, dass eine zentrale Pumpeneinheit, insbesondere eine zentrale Mikropumpe, mit einer Abgabeeinheit verbunden ist, wobei die Pumpeneinheit mehrere Zuflussleitungen aufweist und über jede Zuflussleitung ein unterschiedlicher Duftstoff zugeführt wird. Die Zuführleitungen können einen unterschiedlichen Querschnitt aufweisen, so dass durch die Saugwirkung der Pumpeneinheit, insbesondere der Mikropumpe, unterschiedliche Duftstoffmengen, entweder unmittelbar in die Pumpeneinheit gesaugt werden, oder einer vorgeschalteten Mischkammer zugeführt werden, wobei sich die Duftstoffe vermischen und als Duftstoffkombination abgegeben werden. Rückschlagventile in den einzelnen Zuflussleitungen verhindern eine ungewollte Durchmischung bei deaktivierter Pumpeneinheit. Selbstverständlich können in den einzelnen Zuflussleitungen auch separate Absperr- und Stellorgane vorgesehen sein, um den jeweiligen Zufluss des Duftsstoffes zu der zentralen Pumpeneinheit separat zu steuern oder zu regeln. Eine entsprechend ausgestaltete Vorrichtung kommt selbstverständlich nur dann in Betracht, wenn die sich überlagernden Duftstoffe miteinander harmonieren. Dies kann beispielsweise dadurch sichergestellt werden, dass bestimmte Duftstoffspeicher nur an bestimmten Positionen der Vorrichtung anbringbar sind, um ein ungewolltes Vertauschen und eine unerwünschte Einflussnahme auf die Duftstoffzusammensetzung zu verhindern.

Es ist im Rahmen der Erfindung auch vorstellbar, dass die einzelnen Duftstoffspeicher mit einer Kodierung vorgesehen sind, die bei Befestigung an der Vorrichtung von einer entsprechenden Leseeinheit ausgelesen wird. Die Leseeinheit leitet die ausgelesenen Informationen der Steuereinheit zu, welche in Abhängigkeit von der Information die Dosierung des neu hinzugefügten Duftstoffspeichers zu steuert bzw. regelt. Auf diese Weise kann eine Abstimmung der Dosierung auf die Duftstoffe der bereits vorhandenen Duftstoffspeicher erfolgen. Die verwendete Vorrichtung kann zudem die Eigenschaft besitzen, dass die Steuereinheit auf veränderte Bereitstellung von Duftstoffen in der Weise reagiert, dass bei einer Entleerung eines Duftstoffspeichers entweder ein entsprechendes Signal abgegeben wird und/oder die mengenmäßige Duftstoffzusammensetzung in der Weise modifiziert wird, dass die vorhandenen Duftstoffe in einem neuen Mischverhältnis zu einer neuen Duftkombination zusammengefügt werden, um den zeitweisen Verlust eines Duftstoffs in olfaktorisch angenehmer Weise zu kompensieren.

Der gegenständliche Teil der Erfindung wird durch die Vorrichtung mit den Merkmalen des Patentanspruchs 11 gelöst.

Die erfindungsgemäße Vorrichtung zur Durchführung des Verfahrens aus einem der Ansprüche 1 bis 10 umfasst wenigstens eine Zuflussleitung zur Zuführung des Duftstoffs zu wenigstens einer Abgabeeinheit, an welcher der zugeführte Duftstoff unter Einfluss einer elektrischen Ladung in ein Aerosol überführt wird. Sie umfasst des Weiteren eine an die Abgabeeinheit angeschlossene Hochspannungseinheit zur Beaufschlagung des Duftstoffs mit der elektrischen Ladung, eine Steuereinheit und wenigstens ein mit der Steuereinheit verbundenes Absperr- und Regelorgang zum Absperren der Zuflussleitung. Wesentlich ist, dass eine dem Volumenstrom des Duftstoffs beeinflussende Mikropumpe vorgesehen ist, wobei das maximale Fördervolumen der Mikropumpe kleiner als die maximale Ausbringungskapazität der Abgabeeinheit ist, so dass durch zeitversetzte Aktivierung der Hochspannungseinheit und der Mikropumpe einer innerhalb der Zuflussleitung zwischen dem Absperr- und Stellorgang und der Abgabeeinheit vorhandene Duftstoffmenge reduzierbar ist. Bei dieser Vorrichtung ist das Absperr- und Stellorgang vorzugsweise Bestandteil der Mikropumpe, so dass bei einem Abschalten der Mikropumpe die Abgabeeinheit eine vorbestimmte Zeiteinheit weiterbetrieben werden soll und die zwischen der Mikropumpe bzw. dem Absperr- und Stellorgang und der Abgabeeinheit vorhandene Duftstoffmenge verringert wird. Die Verringerung oder Reduzierung erfolgt, wie eingangs beschrieben, durch das Elektrosprayverfahren.

Die erfindungsgemäße Vorrichtung kann mehrere separate Abgabeeinheiten mit jeweils einer Zuflussleitung und jeweils einer Mikropumpe umfassen, wobei über jede Zuflussleitung ein anderer Duftstoff zugeführt wird. Grundsätzlich ist es aber auch möglich, dass von einer Mikropumpe mehrere parallel geschaltete Abgabeeinheiten gespeist werden, falls eine besonders hohe Ausbringungsrate eines Duftstoffs gewünscht ist. Umgekehrt können auch mehrere Zuflussleitungen an eine gemeinsame Mikropumpe angeschlossen sein, wobei über die einzelnen Zuflussleitungen unterschiedliche Duftstoffe zugeführt werden, die über eine gemeinsame Abgabeeinheit in ein Aerosol überführt werden.

Da es sich bei Mikropumpen um ausgesprochen kleine Bauteile handelt, besteht hinsichtlich ihrer räumlichen Anordnung eine hohe Flexibilität. Den Großteil des Volumens der erfindungsgemäßen Vorrichtung wird voraussichtlich von den Dufststoffspeichern eingenommen werden sowie von einer Einrichtung zur Erzeugung eines Luftstroms. Dies kann beispielsweise ein Ventilator sein. In den von dem Ventilator aufgebrachten Luftstrom wird der jeweilige Duftstoff vernebelt.

Die Erfindung wird nachfolgend anhand der in den schematischen Zeichnungen dargestellten Ausführungsbeispiele näher erläutert. Es zeigen:
- Figur 1: den schematischen Aufbau einer Vorrichtung zur Durchführung des Verfahrens zum Verteilen von flüssigen Duftstoffen;
- Figur 2: ein Diagramm zur Verdeutlichung der Schaltzeiten der Abgabeeinheit und des Absperr- und Stellorgans;
- Figur 3: eine erste mögliche Vorrichtung zur Durchführung des Verfahrens;
- Figur 4: eine weitere mögliche Anordnung einer Vorrichtung zur Durchführung des Verfahrens;
- Figur 5: eine Vorrichtung zur Durchführung des Verfahrens mit mehreren Abgabeeinheiten;
- Figur 6: eine Vorrichtung zur Durchführung des Verfahrens mit mehreren Reservoirs;
- Figur 7: eine Ausführungsform mit mehreren parallel geschalteten Pumpen und Reservoirs;
- Figur 8: eine Ausführungsform mit zusätzlichen Absperr- und Stellorgangen sowie mehreren Abgabeeinheiten;
- Figur 9: eine Ausführungsform ähnlich der Figur 8, jedoch nur mit einer Abgabeeinheit.

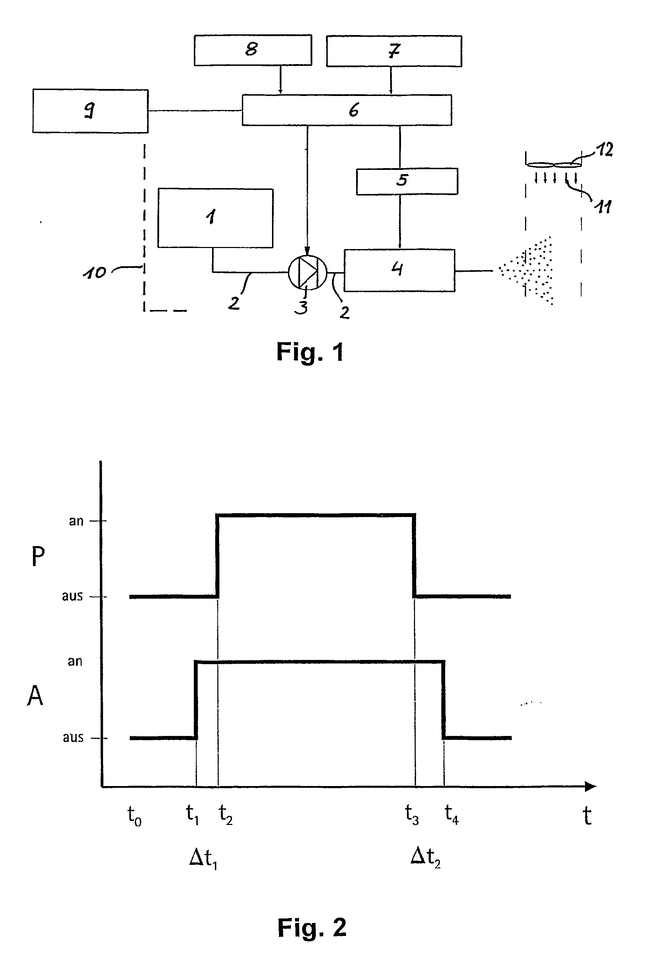

In Figur 1 ist mit 1 ein Duftstoffspeicher bzw. ein Reservoir berechnet, in dem ein Duftstoff bevorratet ist. Über eine Zuflussleitung 2 wird der Duftstoff einer Mikropumpe 3 zugeführt, die gleichzeitig als Absperr- und Stellorgan dient. Die Mikropumpe 3 befördert den Duftstoff zu einer Abgabeeinheit 4, an welcher der Duftstoff unter Einfluss einer elektrischen Ladung in ein Aerosol überführt wird. Eine an die Abgabeeinheit 4 angeschlossene Hochspannungseinheit 5 dient zur Beaufschlagung des Duftstoffs mit der elektrischen Ladung. Es wird eine Gleichspannung in Höhe von 0,5 kV bis 25 kV angelegt. Die Ansteuerung der Hochspannungseinheit 5 und der Mikropumpe 3 erfolgt über eine Steuereinheit 6. Diese ist optional mit einer Schnittstelle 7 zur manuellen Einflussnahme auf die Duftstoffzerstäubung sowie einer zweiten Schnittstelle 8 versehen, die zur Ankopplung an eine externe Steuerung oder ein Programmiermittel dient. Eine Energiequelle 9 zur Versorgung der Vorrichtung mit elektrischer Energie kann in Form einer Batterie in ein andeutungsweise eingezeichntes Gehäuse 10 integriert sein. Es ist auch möglich, dass es sich um eine externe Energiequelle handelt. Die Abgabeeinheit 4 gibt den Duftstoff als feinen Nebel in einen Luftstrom 11 ab, der von Mitteln zur Erzeugung eines Luftstroms 12 aufgebracht wird. Diese Mittel können innerhalb des Gehäuses 10 angeordnet sein. Es ist möglich, dass diese Mittel z.B. Bestandteil einer Klimatisierungsanlage sind.

Figur 2 zeigt den zeitlichen Verlauf der Aktivierung einer mit A bezeichneten Abgabeeinheit und einer mit P bezeichneten Mikropumpe. Zum Zeitpunkt T0 ist weder die Abgabeeinheit A noch die Mikropumpe P aktiviert, d.h. der Volumenstrom innerhalb der Zuflussleitung 2 ist Null. zum Zeitpunkt T1, d.h. mit Einleitung des Elektrosprayvorgangs wird zunächst die Abgabeeinheit A aktiviert. Hierzu wird von der Hochspannungseinheit 5 eine Hochspannung erzeugt und an der Abgabeeinheit A angelegt. Das entspricht einem Wechsel des Schaltzustands der Abgabeeinheit A von "aus" nach "an". Der Volumenstrom ist zu diesem Zeitpunkt noch immer Null. Erst nach einer Zeitdifferenz ΔT1 wird zu einem Zeitpunkt T2 = T1 + ΔT1 die Mikropumpe angeschaltet, so dass Duftstoff durch die Zuflussleitung der Abgabeeinheit A geführt wird. Es schließt sich der Sprayvorgang an, solange ein Duftstoff der Abgabeeinheit A zugeführt wird. Zum Zeitpunkt T3 wird die Mikropumpe P abgeschaltet, während die Abgabeeinheit A noch aktiviert ist. Erst zum Zeitpunkt T4 = T3 + ΔT2 wird auch die Abgabeeinheit A abgeschaltet. Die Differenzen ΔT1 und ΔT2 stellen sicher, dass Duftstoff nicht unkontrolliert abgegeben wird. Eine Bildung großer Tropfen an der Abgabeeinheit wird durch die vorzeitige Abschaltung der Mikropumpe P verhindert.

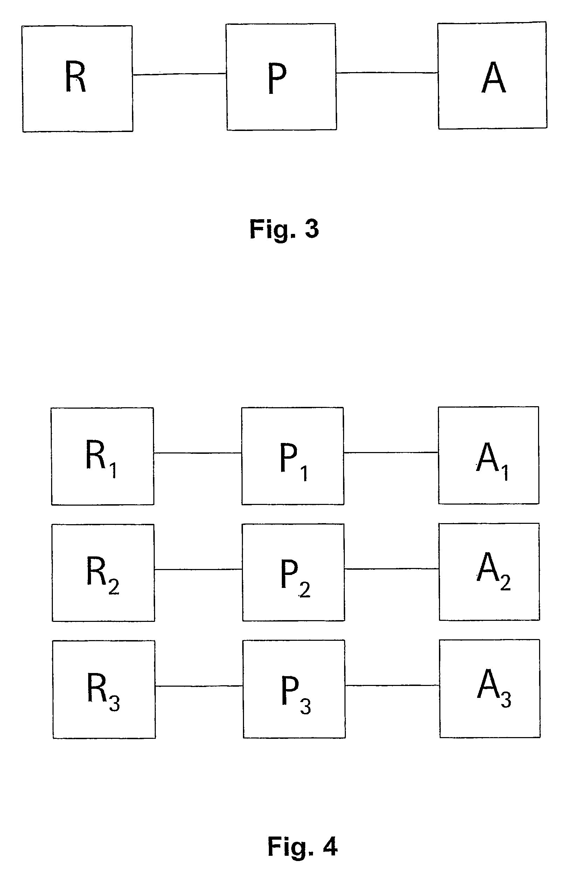

Figur 3 zeigt den grundlegenden Aufbau der erfindungsgemäßen Vorrichtung mit einer Abgabeeinheit A, einer der Abgabeeinheit vorgeschalteten Mikropumpe P als Absperr- und Stellorgan, die einen Duftstoff aus einem Reservoir R entnimmt. Die weiteren Elemente der erfindungsgemäßen Vorrichtung wie die Hochspannungseinheit und die Steuereinheit sind zur Vereinfachung nicht näher dargestellt.

In Abwandlung zu Figur 3 können innerhalb einer Vorrichtung mehrere Abgabeeinheiten A1, A2, A3, sowie wie Mikropumpen P1, P2, P3 und Reservoirs R1, R2, R3 parallel geschaltet sein (Figur 4). Die Anzahl der Parallelschaltungen ist grundsätzlich beliebig.

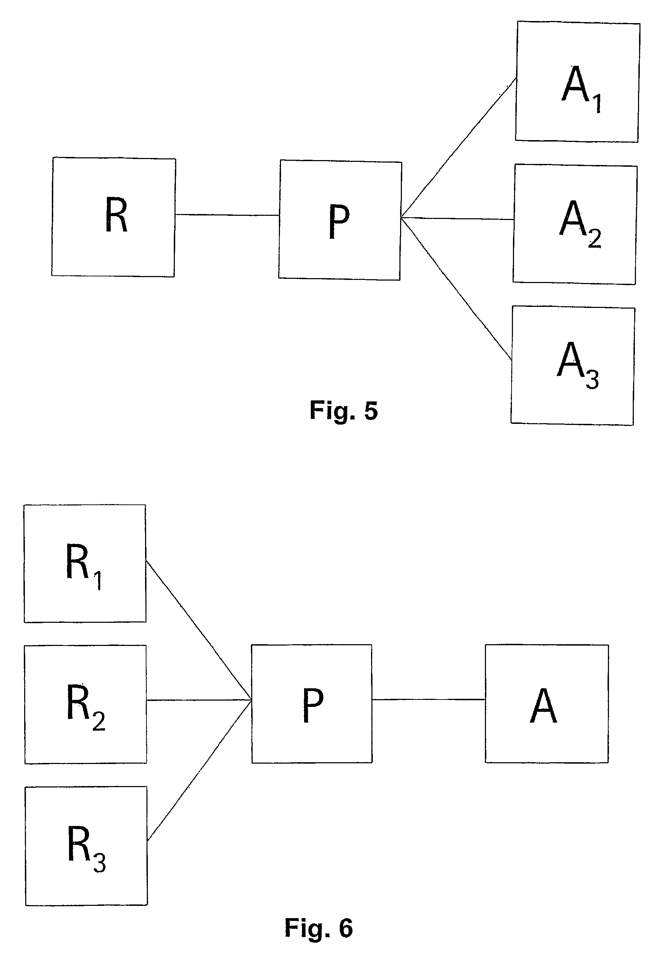

In der Ausgestaltung der Figur 5 ist vorgesehen, dass aus einem einzigen Reservoir R über eine zentrale Mikropumpe P mehrere parallel geschaltete Abgabeeinheiten A1, A2, A3 gespeist werden, um den Duftstoff entweder an unterschiedlichen Orten, d.h. räumlich getrennt abgeben zu können oder um größere Volumenströme an einem einzigen Ort fein verteilt abgeben zu können.

In der Variante der Figur 6 sind mehrere Reservoirs R1, R2, R3 parallel geschaltet. In den Reservoirs R1, R2, R3 können unterschiedliche Duftstoffe bevorratet sein. Eine zentrale Mikropumpe P entnimmt den Reservoirs R1, R2, R3 die gewünschte Menge des Duftstoffs und führt sie einer zentralen Abgabeeinheit A zu.

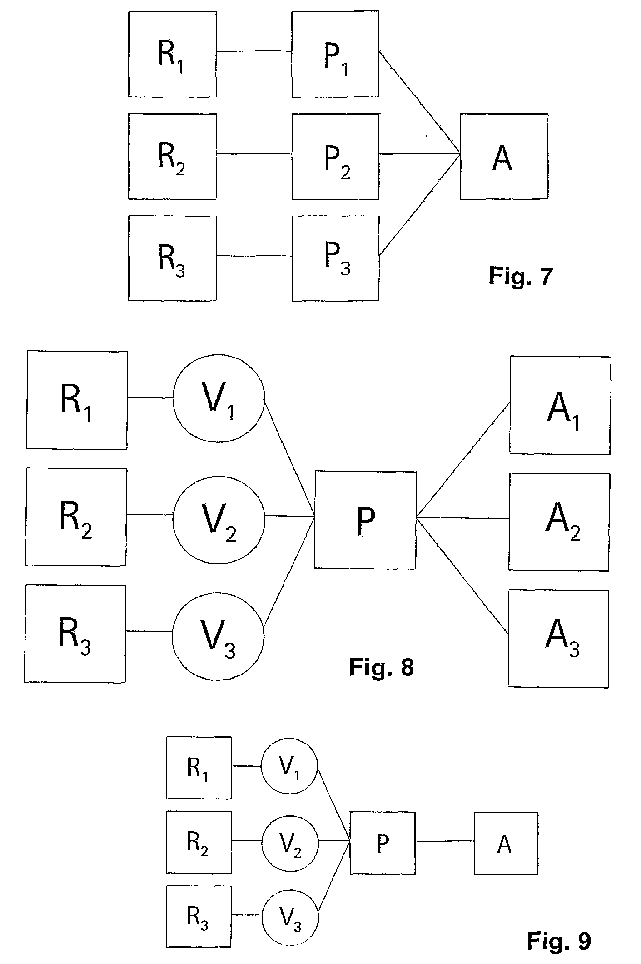

Figur 7 zeigt eine Ausführungsform mit mehreren parallel geschalteten Reservoirs R1, R2, R3, sowie parallel geschalteten Mikropumpen P1, P2, P3, welche die entsprechenden Duftstoffe einer zentralen Abgabeeinheit A zuführen.

Figur 8 zeigt eine Variante, welcher sowohl mehrere Reservoirs R1, R2, R3 als auch mehrere Abgabeeinheiten A1, A2, A3 vorgesehen sind, die von einer zentralen Pumpe P gespeist werden. Im Unterschied zu den vorhergehenden Ausführungsformen ist in der Zuflussleitung zwischen den einzelnen Reservoirs R1, R2, R3 und der zentralen Mikropumpe P jeweils noch ein Ventil V1, V2, V3 als Absperr- und Stellorgan vorgesehen, um die jeweiligen Duftstoffe gezielt den einzelnen Reservoirs R1, R2, R3 durch öffnen oder sperren der Zuflussleitungen entnehmen zu können. Die Ventile V werden ebenfalls von der nicht näher dargestellten Steuereinheit angesteuert.

Figur 9 zeigt schließlich eine Abwandlung der Ausführungsform der Figur 8, die darin besteht, dass Duftstoff nur an einer einzigen zentralen Abgabeeinheit abgegeben wird.

Eine Abgabeeinheit im Sinne der Erfindung kann sowohl eine als auch mehrere mit Hochspannung beaufschlagte Abgaberöhrchen aufweisen, an denen jeweils für sich gesehen der Duftstoff mit einer elektrischen Hochspannung beaufschlagt wird. Mehrere parallel geschaltete Abgabeeinheiten, wie sie in den Figuren 4, 5 und 8 dargestellt sind, können ebenfalls ein oder mehrere derartige Abgaberöhrchen aufweisen.

### Bezugszeichenaufstellung

- 1 -: Duftstoffspeicher
- 2 -: Zuflussleitung
- 3 -: Mikropumpe
- 4 -: Abgabeeinheit
- 5 -: Hochspannungseinheit
- 6 -: Steuereinheit
- 7 -: Schnittstelle
- 8 -: Schnittstelle
- 9 -: Energiequelle
- 10 -: Gehäuse
- 11 -: Luftstrom
- 12 -: Mittel zur Erzeugung eines Luftstroms

- A -: Abgabeeinheit
- P -: Mikropumpe
- R -: Reservoir
- V -: Ventil

## Patentansprüche

1. Verfahren zum Verteilen von flüssigen Duftstoffen, unter Verwendung einer Vorrichtung mit wenigstens einer Zuflussleitung (2) zur Zuführung des Duftstoffs zu wenigstens einer Abgabeeinheit (4, A), an welcher der zugeführte Duftstoff unter Einfluss einer elektrischen Ladung in ein Aerosol überführt wird, einer an die Abgabeeinheit (4, A) angeschlossenen Hochspannungseinheit (5) zur Beaufschlagung des Duftstoffs mit der elektrischer Ladung, einer Steuereinheit (6) und wenigstens einem mit der Steuereinheit (6) verbundenen Absperr- und Stellorgan (3, V, P) zum Absperren der Zuflussleitung (2), **dadurch gekennzeichnet, dass** das Absperr- und Stellorgan (3, V, P) und die Hochspannungseinheit (5) zueinander derart zeitversetzt aktiviert werden, dass eine innerhalb der Zuflussleitung (2) zwischen dem Absperr- und Stellorgan (3, V, P) und der Abgabeeinheit (4, A) vorhandene Duftstoffmenge reduziert wird.

2. Verfahren nach Anspruch 1, **dadurch gekennzeichnet, dass** bei der Verteilung des Duftstoffs zunächst die Hochspannungseinheit (5) aktiviert wird und anschließend das Absperr- und Stellorgan (3, V, P) geöffnet wird.

3. Verfahren nach Anspruch 1 oder 2, **dadurch gekennzeichnet, dass** bei Beendigung und/oder Unterbrechung der Verteilung des Duftstoffes zunächst das Absperr- und Stellorgan (3, V, P) geschlossen und anschließend die Hochspannungseinheit (5) deaktiviert wird.

4. Verfahren nach einem der Ansprüche 1 bis 3, **dadurch gekennzeichnet, dass** an die Abgabeeinheit (4, A) eine elektrische Hochspannung in einem Bereich von 0,5 kV bis 25 kV angelegt wird.

5. Verfahren nach Anspruch 4, **dadurch gekennzeichnet, dass** an die elektrische Hochspannung in einem Bereich von 1,5 kV bis 6 kV liegt.

6. Verfahren nach Anspruch 4 oder 5, **dadurch gekennzeichnet, dass** die elektrische Hochspannung konstant gehalten wird, während der Volumenstrom des Duftstoffs über das Absperr- und Stellorgan (3, V, P) gesteuert wird.

7. Verfahren nach Anspruch 6, **dadurch gekennzeichnet, dass** das Verändern des Volumenstroms mit Hilfe einer Mikropumpe (3, P) durchgeführt wird.

8. Verfahren nach Anspruch 7, **dadurch gekennzeichnet, dass** das maximale Fördervolumen der Mikropumpe (3, P) kleiner oder gleich der Ausbringungskapazität der Abgabeeinheit (4, A) eingestellt wird.

9. Verfahren nach einem der Ansprüche 1 bis 8, **dadurch gekennzeichnet, dass** jeder Abgabeeinheit (A1, A2, A3) ein unterschiedlicher Duftstoff zugeführt wird, wobei die Duftstoffe separat zeit- und/oder volumengesteuert in Aerosole überführt werden.

10. Verfahren nach einem der Ansprüche 1 bis 9, **dadurch gekennzeichnet, dass** der Duftstoff über die Zuflussleitung (2) einem austauschbaren Duftstoffspeicher (1) mit einer flexiblen Umhüllung entnommen wird.

11. Vorrichtung zur Durchführung des Verfahrens nach einem der Ansprüche 1 bis 10, mit wenigstens einer Zuflussleitung (2) zur Zuführung des Duftstoffs zu wenigstens einer Abgabeeinheit (4, A), an welcher der zugeführte Duftstoff unter Einfluss einer elektrischen Ladung in ein Aerosol überführt wird, einer an die Abgabeeinheit (4, A) angeschlossenen Hochspannungseinheit (5) zur Beaufschlagung des Duftstoffs mit der elektrischer Ladung, einer Steuereinheit (6) und wenigstens einem mit der Steuereinheit (6) verbundenen Absperr- und Stellorgan (3, V, P) zum Absperren der Zuflussleitung (2), **dadurch gekennzeichnet, dass** eine den Volumenstroms des Duftstoffs beeinflussende Mikropumpe (3, P) vorgesehen ist, welche dem Absperr- und Stellorgan (3, V, P) entspricht, wobei das maximale Fördervolumen der Mikropumpe (3, P) kleiner als die maximale Ausbringungskapazität der Abgabeeinheit (4,A) ist, so dass durch zeitversetzte Aktivierung der Hochspannungseinheit (5) und der Mikropumpe (3, P) eine innerhalb der Zuflussleitung (2) zwischen dem Absperr- und Stellorgan (3, V, P) und der Abgabeeinheit (4, A) vorhandene Duftstoffmenge reduzierbar ist.

## Claims

1. Method for distributing liquid fragrances, using an apparatus having at least one inflow line (2) for supplying the fragrance to at least one dispensing unit (4, A) at which the supplied fragrance is converted into an aerosol under the influence of an electrical charge, a high-voltage unit (5), which is connected to the dispensing unit (4, A), for applying the electrical charge to the fragrance, a control unit (6), and at least one blocking and actuating element (3, V, P), which is connected to the control unit (6), for blocking the inflow line (2), **characterized in that** the blocking and actuating element (3, V, P) and the high-voltage unit (5) are activated in a manner offset in relation to one another in terms of time in such a way that a quantity of fragrance which is present within the inflow line (2) between the blocking and actuating element (3, V, P) and the dispensing unit (4, A) is reduced.

2. Method according to Claim 1, **characterized in that**, when the fragrance is distributed, first the high-voltage unit (5) is activated and then the blocking and actuating element (3, V, P) is opened.

3. Method according to Claim 1 or 2, **characterized in that**, when the distribution of the fragrance is terminated and/or interrupted, first the blocking and actuating element (3, V, P) is closed and then the high-voltage unit (5) is deactivated.

4. Method according to one of Claims 1 to 3, **characterized in that** a high electrical voltage in a range of from 0.5 kV to 25 kV is applied to the dispensing unit (4, A).

5. Method according to Claim 4, **characterized in that** the high electrical voltage is in a range of from 1.5 kV to 6 kV.

6. Method according to Claim 4 or 5, **characterized in that** the high electrical voltage is kept constant, while the volumetric flow of the fragrance is controlled by means of the blocking and actuating element (3, V, P).

7. Method according to Claim 6, **characterized in that** the volumetric flow is varied with the aid of a micropump (3, P).

8. Method according to Claim 7, **characterized in that** the maximum delivery volume of the micropump (3, P) is set to be less than or equal to the output capacity of the dispensing unit (4, A).

9. Method according to one of Claims 1 to 8, **characterized in that** a different fragrance is supplied to each dispensing unit (A1, A2, A3), the fragrances being converted into aerosols separately controlled in terms of time and/or volume.

10. Method according to one of Claims 1 to 9, **characterized in that** the fragrance is taken from a replaceable fragrance reservoir (1) having a flexible casing by means of the inflow line (2).

11. Apparatus for carrying out the method according to one of Claims 1 to 10, having at least one inflow line (2) for supplying the fragrance to at least one dispensing unit (4, A) at which the supplied fragrance is converted into an aerosol under the influence of an electrical charge, a high-voltage unit (5), which is connected to the dispensing unit (4, A), for applying an electrical charge to the fragrance, a control unit (6), and at least one blocking and actuating element (3, V, P), which is connected to the control unit (6), for blocking the inflow line (2), **characterized in that** a micropump (3, P) is provided which influences the volumetric flow of the fragrance and corresponds to the blocking and actuating element (3, V, P), the maximum delivery volume of the micropump (3, P) being less than the maximum output capacity of the dispensing unit (4, A), so that a quantity of fragrance which is present within the inflow line (2) between the blocking and actuating element (3, V, P) and the dispensing unit (4, A) can be reduced by activating the high-voltage unit (5) and the micropump (3, P) in a manner offset in terms of time.

## Revendications

1. Procédé de distribution de parfums liquides en utilisant un dispositif avec au moins une conduite d'alimentation (2) servant à alimenter en parfum au moins une unité de distribution (4, A), dans laquelle est transporté le parfum amené sous l'influence d'une charge électrique dans un aérosol, en utilisant une unité haute tension (5) raccordée à l'unité de distribution (4, A) servant à alimenter en parfum avec la charge électrique, une unité de commande (6) et au moins un organe d'arrêt et de positionnement (3, V, P) relié à l'unité de commande (6) servant à arrêter la conduite d'alimentation (2), **caractérisé en ce que** l'organe d'arrêt et de positionnement (3, V, P) et l'unité de commande haute tension (5) sont activés en temps différé l'un par rapport à l'autre de telle manière qu'une quantité de parfum présente à l'intérieur de la conduite d'alimentation (2) entre l'organe d'arrêt et de positionnement (3, V, P) et l'unité de distribution (4, A) soit réduite.

2. Procédé selon la revendication 1, **caractérisé en ce que** lors de la distribution du parfum, l'unité haute tension (5) est activée en premier lieu, et ensuite l'organe d'arrêt et de positionnement (3, V, P) est ouvert.

3. Procédé selon les revendications 1 ou 2, **caractérisé en ce qu'**à la fin et/ou en cas d'interruption de la distribution du parfum, l'organe d'arrêt et de positionnement (3, V, P) est d'abord fermé, puis l'unité haute tension (5) est désactivée.

4. Procédé selon l'une quelconque des revendications 1 à 3, **caractérisé en ce qu'**une haute tension électrique de 0,5 kV à 25 kV est appliquée à l'unité de distribution (4, A).

5. Procédé selon la revendication 4, **caractérisé en ce que** la haute tension électrique appliquée est de 1,5 kV à 6 kV.

6. Procédé selon les revendications 4 ou 5, **caractérisé en ce que** la haute tension électrique est maintenue constante pendant que le débit volumétrique du parfum est commandé par l'organe d'arrêt et de positionnement (3, V, P).

7. Procédé selon la revendication 6, **caractérisé en ce que** la modification du débit volumétrique est réalisée à l'aide d'une micropompe (3, P).

8. Procédé selon la revendication 7, **caractérisé en ce que** le volume maximal aspiré de la micropompe (3, P) est réglé pour être inférieur ou égal à la capacité d'extraction de l'unité de distribution (4, A).

9. Procédé selon l'une quelconque des revendications 1 à 8, **caractérisé en ce que** chaque unité de distribution (A1, A2, A3) est alimentée par un parfum différent, les parfums étant transportés en aérosols séparément en fonction du temps et/ou du volume.

10. Procédé selon l'une quelconque des revendications 1 à 9, **caractérisé en ce que** le parfum est prélevé par une conduite d'alimentation (2) d'un réservoir de parfum remplaçable (1) avec une enveloppe flexible.

11. Dispositif pour la mise en oeuvre du procédé selon l'une quelconque des revendications 1 à 10, avec au moins une conduite d'alimentation (2) pour amener le parfum à au moins une unité de distribution (4, A) dans laquelle est transportée le parfum amené sous l'influence d'une charge électrique dans un aérosol, une unité haute tension (5) branchée à l'unité de distribution (4, A) pour alimenter le parfum avec la charge électrique, une unité de commande (6) et au moins un organe d'arrêt et de positionnement (3, V, P) raccordé à l'unité de commande (6) pour arrêter la conduite d'alimentation (2), **caractérisé en ce qu'**une micropompe (3, P) influençant le débit volumétrique du parfum est prévue qui correspond à l'organe d'arrêt et de positionnement (3, V, P), le débit volumétrique maximale de la micropompe (3, P) étant inférieur à la capacité maximale d'extraction de l'unité de distribution (4, A), de sorte que l'activation en temps différé de l'unité haute tension (5) et de la micropompe (3, P) permet de réduire une quantité de parfum présente à l'intérieur de la conduite d'alimentation (2) entre l'organe d'arrêt et de positionnement (3, V, P) et l'unité de distribution (4, A).
